Europäisches Patentamt

European Patent Office

Office européen des brevets

(19)

(11) Publication number: **0 283 606 B1**

## (12) EUROPEAN PATENT SPECIFICATION

(45) Date of publication of patent specification: **15.07.92**

(51) Int. Cl.5: **G01N 33/68**, G01N 33/60, G01N 33/543

(21) Application number: **87302547.2**

(22) Date of filing: **24.03.87**

(54) Method for the detection of pregnancy disorders.

(43) Date of publication of application:
**28.09.88 Bulletin 88/39**

(45) Publication of the grant of the patent:
**15.07.92 Bulletin 92/29**

(84) Designated Contracting States:
**AT BE CH DE ES FR GB GR IT LI NL SE**

(56) References cited:
**EP-A- 0 060 491**
**EP-A- 0 101 603**
**EP-A- 0 141 326**

**CHEMICAL ABSTRACTS, vol.104, no.19, 12 May 1986, Columbus, OH (US); G.THAN et al., p.461, no.166150q**

**L.A. HANSON et al.: "Immunology", 1985, Butterworth & Co.Ltd.; pp.47-48**

**CHEMICAL ABSTRACTS, vol.99, no.7, 15 August 1983, Columbus, OH (US); H.BOHN et al., p.316, no.50851t**

**CHEMICAL ABSTRACTS, vol.95, no.3, 20 July 1981, Columbus, OH (US); H.T.SALEM et al.,**

p.480, no.22529t

(73) Proprietor: **Technion Research & Development Foundation Ltd.**
**Technion City**
**IL-32000 Haifa(IL)**

Proprietor: **Silberman, Michael**
**45 Yotam Str.**
**IL-Haifa(IL)**

(72) Inventor: **Silberman, Michael**
**45 Yotam Street**
**IL-Haifa(IL)**

(74) Representative: **Clifford, Frederick Alan et al**
**MARKS & CLERK 57/60 Lincoln's Inn Fields**
**London WC2A 3LS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a new method for the detection of pregnancy disorders. More particularly, the invention relates to a new method for detection of placental damages at a relatively early stage of pregnancy.

**BACKGROUND OF THE INVENTION**

As known, high risk pregnancies constitute about 10 to 25% of pregnancies. Among the high risk pregnancies disorders the following can be mentioned: diabetes, kidney diseases (such as chronic pyelonephritis, chronic glomerlonephritis and renal insufficiency), heart disease (such as primary pulmonary) hypertension. The known methods for controlling the pregnancy progress, have the disadvantage that they detect the status of pregnancy disorders at a stage when the clinical signs and symptoms are already apparent.

There are several hormone assays suggested to give an indication whether placental function is normal or to predict impending fetal death. The tests most widely used are: urine estriol, urine total estrogens, serum unconjugated estriol and serum placental lactogen. As known, estriol is an estrogenic compound produced by the placenta from precursors derived from fetal adrenal cortex and fetal liver. The conjugated form of estriol is excreted in maternal urine. Serum estriol can be measured either as total estriol or as unconjugated estriol. It usually is measured as unconjugated estriol in order to exclude maternal contribution to the conjugated fraction. Urine estriol can be measured as total estriol or as total estrogens, since estriol normally constitues about 90% of urine total estrogens.

Estriol can be detected by immunoassay as early as the ninth week of gestation. Thereafter, estriol values slowly but steadily increase until the last trimester, when there is a more pronounced increase. Clinical use of estriol measurement is based on the fact that severe acute abnormality of the fetoplacental unit, such as a dead or dying placenta, is manifested either by failure of the estriol level to continue rising or by a sudden marked and sustained decrease in the estriol level. A very recent report (M. Scharf et.al. J.Obstet. Gynec.reprod. Biol., 17 365-75, 1984) concludes that in view of the low correlation between patients with abnormal serum free estriol as the antepartum pathological test, the estriol measurement can not be considered a reliable predicting tool to estimate the actual postpartum state in the pregnancy disorders.

Urine total estrogen was the first test used, since total estrogen can be assayed by standard clinical techniques. However, urine glucose, falsely increases the results and certain other substances such as urobilinogen also may interfere. On the other hand, maternal hypertension, preeclampsia, severe anemia and impaired renal function can decrease urine estrogen or estril secretion considerably. Decrease in the contents may also occur to variable degree in a number of fetuses with severe congenital anomalies. It was also reported that continued bed-rest to the pregnant woman, caused an increase in the estriol excretion values of about 20 to 30% over the levels determined from ambulatory persons.

Specification No. EP-A-141 326 discloses the use of the protein PP20. EP-A-060491 discloses the use of the protein PP16. Chemical Abstract 95:22529t discloses the use of protein PP5. Chemical Abstrast 99:50851 deal with the levels of protein PP13 and PP17 in amniotic fluid during healthy pregnancy. Chemical Abstracts 104:166150q deals with the levels of various placental protein including PP13 in amniotic fluid during healthy pregnancy.

Because of the problems associated with collection of urine or serum estriol specimens and interpretation of the values, as well as the disturbing number of false positive and negative test results, most of the clinical people refrain from correlating these measurements with placental damages. Moreover, all the previous methods did not reveal the pregnancy disorders at an early stage of their appearance, but only when the particular disease was already existent.

SUMMARY OF THE INVENTION

It is an object of the present invention to provide a simple method for the detection of pregnancy disorders at an early stage of their appearance. It is another object of the present invention, to provide a simple method for the detection of pregnancy disorders, said method being of a high sensitivity. It is yet another object of the present invention to provide a simple method for the detection of pregnancy disorders, said method being not influenced by other extraneous factor related thereto. The invention is based on the approach by which antigenic compounds are released from a pathological involved tissue into body fluids, using a particular protein specific to human placenta, and can be subsequently determined. Thus the

2

invention consists of a method for determining pregnancy disorders by analysing a biological fluid sample which comprises the steps of:

(a) labelling by radioactive iodine an antigen consisting of the non-hormonal human-derived placental soluble protein PP-13;

(b) incubating and biological fluid sample with the labelled antigen and a first antibody, highly specific for the placental antigen;

(c) applying to the product obtained in step (b) a second antibody attached to a solid composition, precipitating a complex; and

(d) determining the radioactivity in said complex, thus establishing the extent of pregnancy disorders.

It was surprisingly found that using the above procedure with the particular protein, it is possible to establish an early detection of placental damage in pregnant women. This is in contrast to known prior tests which are claiming to determine the pregnancy disorders only after their existence in an advanced stage of placenta illness.

A soluble protein possessing the above physico-chemical properties, found to be suitable for the present invention, was discovered in the last few years and names PP13. This protein is described in the European Patent Application Number 101,603 (assigned to Behringwerke Aktiengeselschaft). As mentioned in the patent specification, this protein could be useful for the diagnostic purpose of tumors detection of trophoblastic character.

The discovery according to the present invention, to utilize a placental protein for the prediction of occurence of pregnancy disorders is quite surprising, in view of prior art statements which discuss various palcental proteins. stipulating that they are of no value for this purpose (British Journal of Obstetrics and Gynaecology, December 1984, Vol. 91, page 1224). The inventor carried out a number of experiments with various placental proteins which do not possess all the above four physico-chemical properties and found indeed that there are many placental proteins which are indeed not suitable for the present invention although the possess one or two of the above properties. Thus for instance PP-4 has a molecular weight of 35,000 (i.e. as required by the present invention) but its carbohydrate content is 2.4% compared with below 1% required by the present invention and accordingly was found to be unsuitable.

Another placental protein, named PP-9 was described and characterized by H.Bohn et al (New soluble placental tissue proteins; Immunology of Human Placental Proteins, Placental Supplement 4, 1982 Praeger Publishers). Its isoelectric point is given to be between 6.4-6.7 and carbohydrate content 5.5%, which are not according to the present invention. The inventor has found that PP-9 is not suitable for the method being of no value in the prediction of occurence of pregnancy disorders.

The inventor is not yet in a position to explain the theoretical aspects, why only a placental protein possessing all the above four physico-chemical properties, such as PP-13, is suitable for the present method, while other placental proteins are unsuitable. However such theoretical explanations are beyond the scope of the invention.

Among the biological fluid to be analyzed, for the antigen determination according to the present invention, the following shall be mentioned: blood, ammiotic fluid or urine.

**BRIEF DESCRIPTION OF THE DRAWINGS**

Figure 1 presents the graphs correlating the percentage of specific binding versus the concentration of the PP-13 protein,

Figure 2 presents the correlating graph of the $^{125}$I radioactivity versus the PP-13 protein concentration,

Figure 3 presents the cumulative distribution in the sera for four groups of population samples.

**DESCRIPTION OF THE PREFERRED EMBODIMENTS**

The first step of labelling the protein by radioactive iodine, is known in the art by the term radioimmunoassay. As known, the radioimmunoassay is based on the competition of a known excess amount of radiolabelled antigen and an unknown variable amount of the unlabelled antigen, for binding sites of a constant amount of antibody. The specific particular method selected for labelling the antigen, is the so called solid phase antibody. This labelling was carried out according to the method of Marchalonis (Biochemical Journal, Volume 113, 1963) with Lactoperoxidase-$H_2O_2$. Due to the alkalinity of the mixture, some phosphate buffer saline, was also added so that the mixture reached a neutral point. The use of radioactive isotopes has enabled the immune reaction to be more easily observed and thus it is preferably to be utilized in the present invention, wherein the specific protein can be determined with great sensitivity.

The radioactive iodine bounded by the antigen is counted, using a Gamma Counter (such as Auto Gamma produced by Packard) and the yield thereto is calculated. The yield is calculated using the following formula:

$$\text{Yield} = \frac{\text{radioactivity measured by the peak of protein}}{\text{Total radioactivity}} \times 100$$

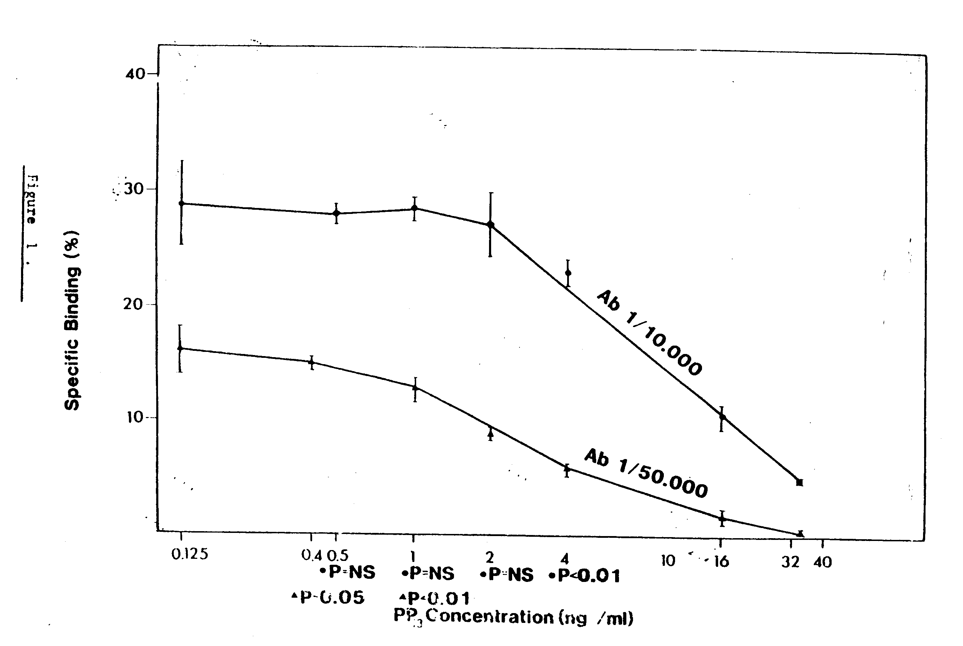

The most preferred concentrations of the antibody for obtaining the proper complex (AG - AB) is between 1/10,000 to 1/50,000. In this range it was discovered that there is a linear correlation between the specific binding and antibody concentration. This appears in a clear manner in Figure 1, wherein the specific binding (expressed in percentage) is given versus the concentration of the PP-13 protein. Based on the yield of the radioactive iodine, the specific activity of the labelled antigen is calculated and subsequently this is correlated with standard evaluation curves.

In the second step, the labelled antigen obtained in step (a) is correlated with the biological fluid (blood, amniotic fluid or urine) and a first antibody. This antibody was obtained from antiserum of the placental antigen in a rabbit, diluted by a buffer (pH = 7.4) consisting of $NaN_3$ (1 g/l) and TRIS - HCl (0.02 mol/l).

In the third step, a second antibody, attached to a solid support, developped in the serum of a donkey, was admixed with the product of step (b) and the precipitated complex was separated by centrifugation and washed with a Phosphate Buffer Saline solution.

Finally, in the fourth step, the radioactivity was determined in the washed complex by an Auto Gamma (produced by Packard).

The above steps of the method will enable to obtain the following data:

(1) The degree level of total radioactivity introduced in the system of the experiment;

(2) The degree of non-specific binding of the labelled antigen and the second antibody;

(3) The zero binding of the antigen and its specific antibody;

(4) Calibration of the system by utilizing a series of known concentrations of the unlabelled antigen.

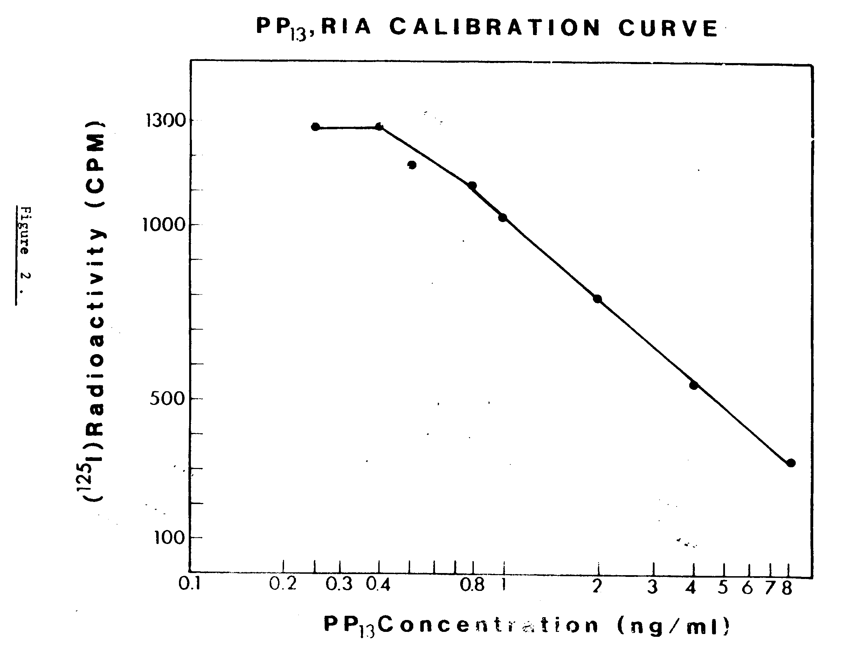

In the attached Figure 2, it is presented the radioimmunoassay calibration curve of the protein, correlating the $^{125}$I radioactivity (expressed in counts per minute) versus the PP-13 protein concentration (expressed in ng/ml).

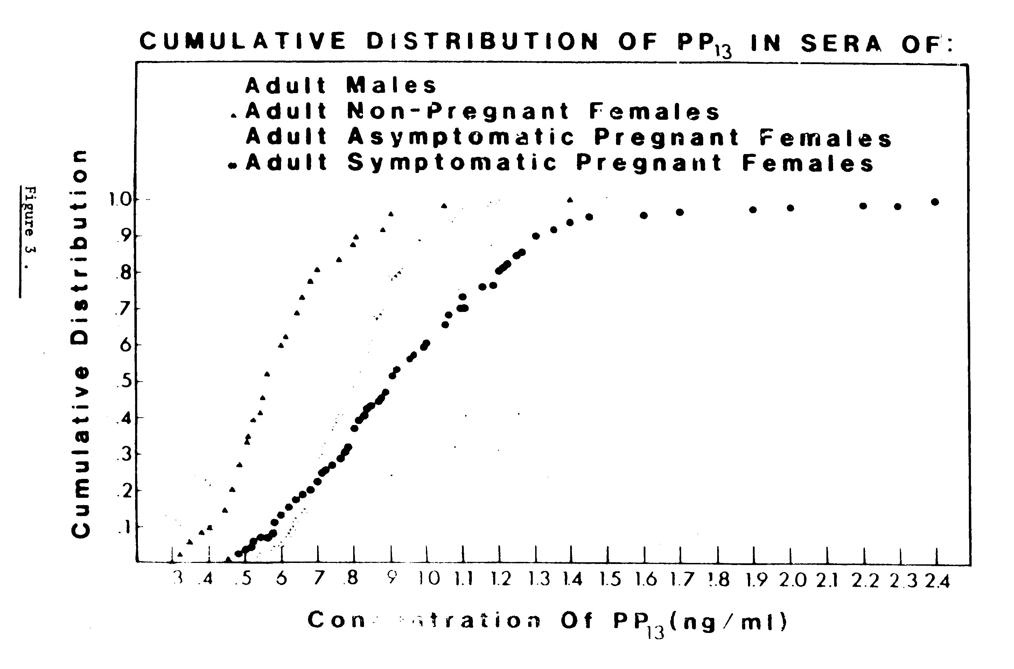

The method according to the present invention was applied to four groups of population samples: (1) adults males; (2) adults non-pregnant females; (3) adult asymptomatic pregnant females; and (4) adult symptomatic pregnant females. The same procedure, with exactly the same reagents and amounts thereof, was employed for all four groups and the results obtained are illustrated in the attached Figure 3. In this Figure, there are correlated the cumulative distribution in the sera for the above four groups versus the concentration of the PP-13 protein. From the graphs presented therein it can be noticed that the group of pregnant females differ from the group of males as well as the group of non-pregnant females. It can be also noticed that, there is a substantially equal concentration of protein for the adult males and non-pregnant females at the same cumulative distribution. However, there is a sharp difference in the protein concentration between asymptomatic pregnant females and symptomatic pregnant females, this concentration being substantially higher in the latter gorup than in the former group particularly at a cumulative distribution of above 0.4, the gap being particularly sensible when the protein concentration is above 0.7 ng/ml. The interpretation method is according to Siegel S (Non-parametric statistics for the behavioural Sciences, Mc.Graw-Hill, 1956, New York).

In the following Table 1 are presented some statistical vlaues concerning the distributional pattern of the PP-13 protein taken as antigen, determined with the method according to the present invention, for the above four groups of population samples. The protein concentration in the blood samples tested was in the range of 0.3 - 2.4 ng/ml.

4

## TABLE 1.

### Statistics of the distribution of the PP-13 protein tested in sera of males, nonpregnant females, healthy pregnant females and nonhealthy pregnant females.

|  | Males | Nonpregnant | Pregnant females | |
|---|---|---|---|---|
|  |  |  | Healthy | Nonhealthy |
| Number | 18 | 48 | 92 | 150 |
| Mean (ng/ml) | 0.58 | 0.61 | 0.82 | 0.95 |
| Standard deviation | 0.14 | 0.20 | 0.16 | 0.34 |
| 1st Quartile | 0.46 | 0.48 | 0.71 | 0.71 |
| 2nd Quartile | 0.58 | 0.56 | 0.82 | 0.90 |
| 3rd Quartile | 0.67 | 0.68 | 0.90 | 1.15 |

From the results presented in the above Table 1, it can be noticed that the protein level is substantially the same in the blood samples of males and non-pregnant females. The level of protein appears to be higher in pregnant females, where in about 75% of the pregnant females there is a protein level of above 0.71 ng/ml. This difference which appears in the Table is significant, from a statistical point of view (P = 0.0035) using the Mann-Whitney criterium. It can also be noticed that in about 50% of nonhealthy pregnant females (compared with about 25% of healthy pregnant females) the value of protein is higher than 0.9 ng/ml.

According to another embodiment of the present invention, the placental protein possessing the above mentioned four physicochemical properties, such as PP-13 can be utilized not only for diagnostic but also for monitoring the pregnancy development and occurrency of placental damage. The method can reveal any abnormal development due to medical treatement for a pregnant female such as diabetes, anemia, hypertension, rhenal, thyroid etc. Based on the results, the physician will decide whether the pregnancy should be ceased, or to change the dose of the medicine, or even to change completely the medicine taken by the pregnant female.

Thus the pregnancy monitoring by the method according to the present invention will be of high importance, being most appreciated by a person versed in the art. This is a most surprising finding, in view of prior statements which conclude that placental proteins could not be specific to pregnancy in view of the differences which exist between them.

While the invention will be hereafter described in a detailed manner with certain preferred embodiments and compositions, it will be understood that it is not intended to limit the invention to these particular embodiments. On the contrary, it is intended to cover other alternatives, modifications or other ingredients as may be included within the scope of the invention as defined by the appended Claims. It should be understood that the particulars are by way of example and for purposes of illustrative discussion of the procedure for carrying out the method according to the present invention, without being limited thereto.

The reagents utilized in the following Examples and the source are as follows:
- Protein PP-13, produced by Behringwerke Aktiengesellschaft, Lot No. 211/233.
- Anti PP-13, antiserum, Lot No. 160 Z B.
- Radioactive iodine ($^{125}$I) produced by Israel Nuclear Center in a solution of Sodium hydroxide (pH = 8-11) possessing a specific activity of 15-20 $\mu$ci/$\mu$g.
- Lactoperoxidase, produced by Sigma (St. Louis, Missouri) Lot No. 2005.
- Hydrogen Peroxide, 30% concentration, produced by Sigma, Lot No. H-1009.

The instruments utilized for the measurements were as follows:

- Gamma Counter, produced by Elscint, Israel (Integrated Nuclear Spectrometer, model INS-11N).
- Auto Gamma 400 (GD, produced by Packard (U.S.A.).
- Cooled Ultra Centrifuge, produced by Cryofuge, Heraeus, West-Germany.
- Column Sephadex (registered trade mark) G-100 (9 x 550 mm).
- Fraction Collector, produced by Pharmacia, Frac-100 (Sweden).

## Step a

The labelling was done by radioactive iodine ($^{125}$I), at room temperature in a 2 ml plastic test tube, having at its bottom a metal rod operated by a magnetic stirrer, which rotates at 2 rotations per seconds. The following reagents were introduced:
- 3 $\mu$g of placental protein in 10 $\mu$l of phosphate buffer saline (pH = 7.2);
- 1 $\mu$g of lactoperoxidose in 6 $\mu$l of phosphate buffer saline;
- 325-350 $\mu$ci of $^{125}$I in 2 $\mu$l of NaOH (pH = 8-11); and
- 9 nmole of hydrogen peroxide in 10 $\mu$l of phosphate buffer saline.

The reaction solution consisted of 5 $\mu$g of PP-13 to which it was added 1 $\mu$g of lactoperoxidase and 325 $\mu$ci of $^{125}$I. Due to the relative high alkalinity prevailing thereto, an amount of 3 $\mu$l of phosphate buffer solution (0.4 M) was added the pH reaching a neutral value. The iodination reaction started by the addition of hydrogen peroxide and continued for about 3 minutes. The iodination was stopped by adding 300 $\mu$l of a solution consisting of: phosphate buffer saline contianing 10 g/l of bovine serum albumen; NaI (2 g/l); NaN$_3$ (1 g/l) and NaCl (8.5 g/l). After an additional stirring for 1 minute, the mixture was passed over a Sephadex (registered trade mark) G-100 column for extracting out the labelled protein from the free iodine and the other reagents, by a solution consiting of: phosphate buffer saline + 1% bovine serum albumen and 0.1% NaN$_3$. From the resulted fractions the extent of iodination was determined by a Gamma Counter (Elscint) and the calculated yield was found to be 64.6%.

In order to calculate the specific activity of the labelled antigen, samples of 10 $\mu$l were taken and again counted by Packard Gamma Counter and found to be 86178 cpm/ng.

## Step b.

Standard solutions of various concentrations in the range of 0.2 ng/ml to 64 ng/ml of antigen in a buffer (tris HCl, pH = 7.4, 0.02 m/l and NaN$_3$, 1 g/l) were prepared.
The labelled antigen consisted of a diluted solution of 1 ng/ml. The first antibody, was the antiserum of the placental antigen diluted in a buffer (with the same composition as above).

## Step c.

The second antibody, was developed in the serum of a donkey. The test was carried out in a plastic test tube, the working scheme being described in the following Table 2.

**TABLE 2.**

<u>The working scheme for the determination of placental</u>

<u>antigen in the human serum using radioimmunoassay.</u>

| Kind of incubate | Buffer A: Tris-HCl, pH=7.4(0.02m/1)+ NaN (1 g/1) 3 (μl) | Standards (μl) | Sample (μl) | Tracer antigen (μl) | First antibody (μl) | Second antibody (μl) |
|---|---|---|---|---|---|---|
| Total Radioactivity | 200 | – | – | 100 | – | – |
| Nonspecific binding | 200 | – | – | 100 | – | 500 |
| Zero binding | 100 | – | – | 100 | 100 | 500 |
| Standards | – | 100 | – | 100 | 100 | 500 |
| Samples | – | – | 100 | 100 | 100 | 500 |

The reactions between the labelled antigen with the sample of serum or respective standards were carried out for about 18 hours are followed by the addition of the second antibody and agitated for about 10 minutes. The mixtures were centrifuged at room temperature for 30 minutes (at 1000 x C) and the precipitates washed with 500 μl of phosphate buffer saline and again centrifuged.

The radioactivity in the washed precipitate was determined by Packard Gamma Counter. The results obtained are summarized in the attached Figure 1. It clearly appears from said Figure that an antibody (A b) with a concentration of 1/50000, gave an adequate binding range for an antigen concentration of between 0.4-32 ng/ml.

In order to calculate the antigen concentrations in the samples tested, a calibration curve was prepared (shown in Figure 2) correlating the radioactivity of $^{125}$I (expressed in counts per minute) versus the PP-13 concentration (expressed in ng/ml).

The results obtained according to the method described in this Example, which were obtained on four groups of population samples: adults males, adults non-pregnant females, adult asymptomatic pregnant females and adult symptomatic pregnant females are illustrated in a graphic manner in Figure 3.

### EXAMPLE 2

Using the same reagents, procedures and working scheme as described in Steps a,b,c and Table 2, seven samples of clear amniotic fluid were analyzed.

The results of these determinations are summarized in Table 3.

<u>Table 3</u>

### <u>Concentration of PP-13 in Human Amniotic Fluid</u>

| Sample No. | Concentration of PP-13 (ng/ml) |
|------------|--------------------------------|
| 1 | 1.24 |
| 2 | 2.99 |
| 3 | 3.66 |
| 4 | 2.07 |
| 5 | 3.13 |
| 6 | 1.57 |
| 7 | 2.86 |

Based on the above results, graphs were drawn correlating the concentration of the PP-13 versus the specific binding (expressed in percentage) and the $^{125}I$ radioactivity (expressed in counte per minute). These graphs were identical as those obtained with serum as illustrated in Figures 1 and 2 based on Example 1, where human serum was analyzed.

## Claims

1. A method for determining pregnancy disorders by analysing a biological fluid sample, which comprises the steps of:
   (a) labelling by radioactive iodine an antigen consisting of a non-hormonal human-derived placental soluble protein;
   (b) incubating said biological fluid sample with the labelled antigen and a first antibody, highly specific for the placental antigen;
   (c) applying to the product obtained in step (b) a second antibody attached to a solid composition, precipitating a complex; and
   (d) determining the radioactivity in said complex characterised in that the non-hormonal human-derived protein is PP13.

2. A method according to claim 1, wherein said biological fluid is selected from the group consisting of blood, amniotic fluid and urine.

3. A method according to claim 1 or 2, wherein a level of said placental protein antigen concentrates above 0.71 mg/ml establishes the occurence of placental damage.

4. A method according to claim 1, wherein the labelling of the antigen involves the following steps:
   (a) extracting the labelled antigen using a solid adsorbent;
   (b) counting the radioactive iodine bounded specifically by the antigen and the yield thereto;
   (c) calculating the specific activity of the labelled antigen; and
   (d) correlating said specific activity with standard evaluation curves.

5. A method according to claim 1, wherein the first antibody is obtained from antiserum of the placental antigen in an animal.

6. A method according to claim 5, wherein said antiserum is diluted by a buffer at a pH of about 7.4.

7. A method according to claim 6, wherein said buffer consists of NaN and TRIS-HC1.

8. A method according to claims 5 to 7, wherein the ratio of the first antibody to the antigen, for obtaining the proper complex AG-AB, is between 1/10,000 to 1/50,000.

**Revendications**

1. Un procédé pour la détection de troubles de grossesse par l'analyse d'un échantillon de fluide biologique, comprenant les étapes suivantes:

(a) marquage à l'iode radioactif d'un antigène conposé d'une protéine placentaire soluble non hormonale dérivée de la protéine humaine;

(b) incubation du dit échantillon de fluide biologique avec l'antigène marqué et un premier anticorps, hautement spécifique de l'antigène placentaire;

(c) application au produit obtenu au cours de l'étape (b) d'un deuxième anticorps fixé à une composition solide, et précipitation d'un complexe; et

(d) détermination de la radioactivité dans ledit complexe, caractérisé en ce que la protéine non hormonale dérivée de la protéine humaine est la PP-13.

2. Un procédé selon la revendication 1, dans lequel ledit fluide biologique est choisi dans le groupe constitué de sang, de liquide amniotique et d'urine.

3. Un procédé selon la revendication 1 ou 2, dans lequel un niveau des concentrations du dit antigène de la protéine placentaire supérieures à 0,71 mg/ml indiquent l'apparition d'une lésion placentaire.

4. Un procédé selon la revendication 1, dans lequel le marquage de l'antigène englobe les étapes suivantes:

(a) extraction de l'antigène marqué avec utilisation d'un absorbant solide;

(b) comptage de l'iode radioactif lié spécifiquement par l'antigène et du rendement correspondant;

(c) calcul de l'activité spécifique de l'antigène marqué; et

(d) mise en corrélation de ladite activité spécifique avec des courbes d'évaluation standards.

5. Un procédé selon la revendication 1, dans lequel le premier anticorps est obtenu à partir de l'antisérum de l'antigène placentaire d'un animal.

6. Un procédé selon la revendication 5, dans lequel ledit antisérum est dilué par un tampon d'un pH d'environ 7,4.

7. Un procédé selon la revendication 6, dans lequel ledit tampon est composé de NaN et de TRIS-HCl.

8. Un procédé selon les revendications 5 à 7, dans lequel le rapport entre le premier anticorps et l'antigène, en vue de l'obtention du complexe AG-AB approprié, est compris entre 1/10.000 et 1/50.000.

**Patentansprüche**

1. Verfahren zur Feststellung von Schwangerschaftsstörungen durch Analyse einer biologischen Flüssigkeitsprobe, welches folgende Schritte umfaßt:

(a) Markierung eines Antigens, das aus einem nichthormonellen, plazentaren, löslichen Protein menschlichen Ursprungs besteht, mit radioaktivem Jod;

(b) Inkubation dieser biologischen Flüssigkeitsprobe mit dem markierten Antigen und einem ersten Antikörper, der für das plazentare Antigen äußerst spezifisch ist;

(c) Aufbringen eines zweiten Antikörpers, der an einer festen Zusammensetzung haftet, auf das in Schritt (b) erhaltene Produkt, Ausfällen eines Komplexes; und

(d) Bestimmung der Radioaktivität in diesem Komplex, dadurch gekennzeichnet, daß das nichthormonelle Protein menschlichen Ursprungs PP13 ist.

2. Verfahren nach Anspruch 1, worin die biologische Flüssigkeit aus der Gruppe, bestehend aus Blut, Fruchtwasser und Urin, gewählt wird.

3. Verfahren nach Anspruch 1 oder 2, worin ein Spiegel dieser plazentaren Protein-Antigenkonzentrate über 0,71 mg/ml eine plazentare Schädigung anzeigt.

4. Verfahren nach Anspruch 1, worin die Markierung des Antigens folgende Schritte beinhaltet:
    (a) Extraktion des markierten Antigens unter Verwendung eines festen Adsorptionsmittels;
    (b) Bestimmung des spezifisch von dem Antigen gebundenen radioaktiven Jods und der Ausbeute;
    (c) Berechnung der spezifischen Aktivität des markierten Antigens; und
    (d) Korrelierung dieser spezifischen Aktivität mit Standard-Auswertungskurven.

5. Verfahren nach Anspruch 1, worin der erste Antikörper von einem Antiserum des plazentaren Antigens eines Tiers erhalten wird.

6. Verfahren nach Anspruch 5, worin das Antiserum mit einem Puffer bei einem pH von etwa 7,4 verdünnt wird.

7. Verfahren nach Anspruch 6, worin der Puffer aus NaN und TRIS-HCl besteht.

8. Verfahren nach den Ansprüchen 5 bis 7, worin zum Erhalt des richtigen AG-AB-Komplexes das Verhältnis des ersten Antikörpers zu dem Antigen zwischen 1/10.000 und 1/50.000 liegt.

Figure 1.

EP 0 283 606 B1

## PP$_{13}$, RIA CALIBRATION CURVE

Figure 2.

EP 0 283 606 B1

CUMULATIVE DISTRIBUTION OF PP$_{13}$ IN SERA OF:

Adult Males
Adult Non-Pregnant Females
Adult Asymptomatic Pregnant Females
Adult Symptomatic Pregnant Females

Cumulative Distribution

Concentration Of PP$_{13}$(ng/ml)

Figure 3 .

EP 0 283 606 B1